(19) Europäisches Patentamt
European Patent Office
Office européen des brevets

(11) Publication number: **0 106 487**
**A2**

(12) # EUROPEAN PATENT APPLICATION

(21) Application number: 83305133.7

(22) Date of filing: 05.09.83

(51) Int. Cl.[3]: C 07 D 221/22
C 07 D 407/06, C 07 D 409/06
A 61 K 31/47
//C07C43/23, C07C43/215,
C07D221/10

(30) Priority: 07.09.82 US 415499

(43) Date of publication of application:
25.04.84 Bulletin 84/17

(84) Designated Contracting States:
AT BE CH DE FR GB IT LI LU NL SE

(71) Applicant: ELI LILLY AND COMPANY
307, East McCarty Street
Indianapolis Indiana 46285(US)

(72) Inventor: Zimmerman, Dennis Michael
131 Thorncrest Drive
Mooresville Indiana 46158(US)

(74) Representative: Crowther, Terence Roger et al,
Erl Wood Manor
Windlesham Surrey GU20 6PH(GB)

(54) **Improvements in or relating to novel bridged hexahydrobenz(f)isoquinolines.**

(57) It has been found that novel bridged hexahydrobenz[f]isoquinoline derivatives, or a pharmaceutically-acceptable salt thereof, are useful analgesic agents, having both narcotic agonist and antagonist properties, as well as psychotropic characteristics.

EP 0 106 487 A2

# IMPROVEMENTS IN OR RELATING TO NOVEL BRIDGED HEXAHYDROBENZ[f]ISOQUINOLINES

It has long been known that slight chemical modifications of the morphine molecule lead to analgesic agonists of widely differing potencies and addictive properties. For example, codeine, the methyl ether of morphine, is a relatively mild analgesic agonist having only slight dependance (addiction) probabilities. On the other hand, heroin, the diacetyl derivative of morphine, is a powerful agonist with extremely high addiction potential. In addition, as long ago as 1915, Pohl found that when the N-methyl group of codeine was replaced with an allyl group, the resulting compound, N-allylnorcodeine, was an opiate antagonist. In 1940, N-allylnormorphine or nalorphine was synthesized and was shown to have a highly specific ability to reverse the depressant effects of morphine. Other simple chemical modifications of the morphine molecule have yielded many interesting drugs. Thus, one fruitful research area in the search for improved analgesics of high potency or lower addiction potential has been the chemical modification of the morphine molecule.

In addition to chemically modifying the morphine ring structure, chemists have developed a second related field of research--the preparation of compounds containing only part-structures of morphine--with the same end in mind as above; i.e., the synthesis of improved analgesic agonists and/or analgesic antagonists of improved properties. For example, meperidine,

a widely used analgesic, can be written as a morphine part-structure, as can the benzomorphans and the prodines. Many other compounds of morphine part-structures have been prepared, some of which have improved analgesic agonist properties and others, particularly those with an allyl group attached to a ring nitrogen, which have opiate antagonist properties. It had been hoped that morphine part-structure research would produce a compound having both opiate agonist and antagonist properties since the opiate antagonist property would assure a user that the compound would have a greatly reduced dependance liability. Two recently marketed analgesics, pentazocine and phenazocine, have been found to be both antagonists and agonists although they still retain a certain degree of opiate dependance liability.

Recently, two groups have prepared isoquinoline analogs of the prodines and/or the benzomorphans. Menard, et al., Can. J. Chem., 52, 2316 (1974), prepared several octahydrobenz[f]isoquinolines which were reported to possess analgesic activity. All of the compounds prepared and tested were substituted at the 10b position with hydroxy or acyloxy.

Hahne, et al., Arch. Pharm., 312, 472 (1979), reported the preparation and analgesic activity of octahydrobenz[f]isoquinolines which are not substituted in the benzene portion of the molecule.

In accordance with the present invention, novel bridged hexahydrobenz[f]isoquinoline derivatives are useful as analgesic agents, having both narcotic agonist and antagonist properties, as well as psychotropic characteristics.

Accordingly, compounds of formula (I),

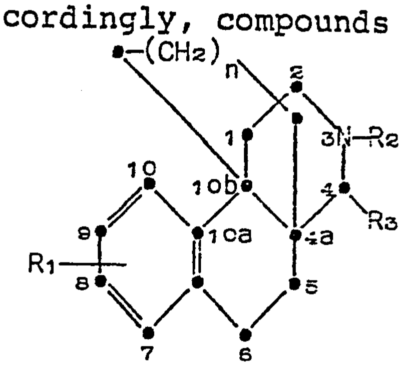

I

or a pharmaceutically-acceptable salt thereof, in which

$R_1$ is hydrogen, hydroxy, $C_1$-$C_4$ alkoxy, or $C_1$-$C_{12}$ alkanoyloxy;

$R_2$ is hydrogen, $C_1$-$C_8$ alkyl, $C_2$-$C_6$ alkenyl-methyl, ($C_3$-$C_8$ cycloalkyl)methyl,

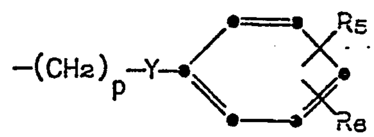

, or

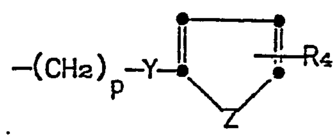

,

in which p is 0, 1, or 2;

Y is $-\overset{O}{\overset{\|}{C}}-$, $-\overset{OH}{\overset{|}{C}H}-$, $-CH_2-$, -O-, -S-, or -NH-; providing that when Y is -O-, -S-, or -NH-, p may only be 2, and providing that when Y is $-\overset{OH}{\overset{|}{C}H}-$, p may not be 0;

Z is O or S;

$R_5$ is $C_1$-$C_4$ alkylthio, nitro, amino, trifluoromethyl, hydroxy, hydrogen, $C_1$-$C_4$ alkyl, $C_1$-$C_4$ alkoxy, or halo;

$R_6$ is hydrogen, $C_1$-$C_4$ alkyl, $C_1$-$C_4$ alkoxy, or halo;

$R_4$ is hydrogen or methyl;

$R_3$ is $C_1$-$C_8$ alkyl or hydrogen; and

n is 1 or 2, are useful analgesic and psychotropic agents.

In a further aspect of the invention, there is provided a process for preparing the compounds of Formula (I) which comprises

(a) Reducing a compound of Formula (II),

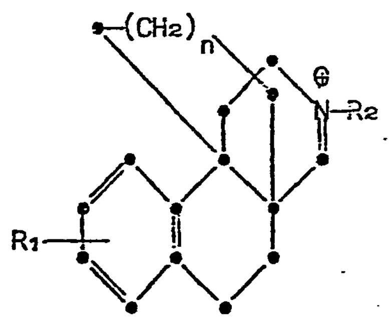

(II)

to form a compound of Formula (I) in which $R_3$ is hydrogen; or,

(b) Reacting a compound of Formula (II) of step (a), in which $R_1$ is hydrogen or $C_1$-$C_4$ alkoxy, with an organometallic reagent to form a compound of Formula (I) in which $R_3$ is $C_1$-$C_8$ alkyl; or,

(c) Hydrolysis or reduction of a compound of Formula (I) in which $R_1$ is $C_1$-$C_4$ alkoxy or $C_1$-$C_{12}$ alkanoyloxy, to form a compound in which $R_1$ is hydroxy; or,

(d) Acylation or alkylation of a compound of Formula (I) in which $R_1$ is hydroxy to form a compound of Formula (I) in which $R_1$ is alkanoyloxy or alkoxy; or,

(e) Treatment of a compound of Formula (I), in which $R_2$ is $-\underset{\underset{O}{\|}}{C}-OW$ where W is $C_1$-$C_4$ alkyl, benzyl or phenyl, with a base to form a compound of Formula (I) in which $R_2$ is hydrogen; or,

(f) Reacting a compound of Formula (I) in which $R_2$ is hydrogen, with an alkyl halide, or acyl halide followed by reduction, to form a compound of Formula (I) in which $R_2$ is other than hydrogen; and,

(g) if desired, salifying a product of any of reactions (a)-(f).

The term "$C_1$-$C_8$ alkyl" refers to straight and branched alkyl radicals of one to eight carbon atoms and includes methyl, ethyl, n-propyl, isopropyl, tert-butyl, hexyl, octyl, and includes the terms "$C_1$-$C_4$ alkyl" and "$C_2$-$C_4$ alkyl". The term "$C_2$-$C_6$ alkenylmethyl" refers to straight and branched alkene radicals of two to six carbon atoms attached to a methyl radical, such as 2-propenyl (allyl), 2-butenyl, 3-butenyl, or 2-pentenyl. The term "($C_3$-$C_8$ cycloalkyl)methyl" refers to a methyl radical substituted with saturated alicyclic rings of three to eight carbon atoms, such as cyclopropylmethyl, methylcyclopropylmethyl, cyclobutylmethyl, cyclopentylmethyl, cyclohexylmethyl, 1-, 2-, 3-, or 4-methylcyclohexylmethyl, cycloheptylmethyl, or cyclooctylmethyl.

The term "$C_1$-$C_4$ alkoxy" refers to the straight and branched aliphatic ether radicals of one to four carbon atoms such as methoxy, ethoxy, propoxy, isopropoxy, butoxy, isobutoxy, sec-butoxy, or tert-butoxy, and includes the term "$C_2$-$C_4$ alkoxy".

The term "$C_1$-$C_{12}$ alkanoyloxy" refers to straight and branched aliphatic acyloxy radicals of one to twelve carbon atoms, such as formoyl, acetoxy, propionyloxy, pentanoyloxy, 4-butyloctanoyloxy, and lauroyloxy.

The term "halo" refers to fluoro, chloro, bromo, and iodo.

The pharmaceutically-acceptable salts, including quaternary ammonium salts, of the amine bases represented by the above formula are formed with acids, and include, for example, salts derived from inorganic acids such as hydrochloric acid, nitric acid, phosphoric acid, sulfuric acid, hydrobromic acid, hydroiodic acid, nitrous acid, or phosphorous acid, as well as salts derived from organic acids including aliphatic mono- and dicarboxylic acids, phenyl-substituted alkanoic acids, hydroxy alkanoic and alkanedioic acids, aromatic acids, aliphatic and aromatic sulfonic acids. Such pharmaceutically-acceptable salts may include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate,

suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate, or naphthalene-2-sulfonate.

"Pharmaceutically-acceptable salts" are those salts useful in the chemotherapy of warm-blooded animals.

The usual nonreactive solvents used in the preparation of the salt from the base include, for example, acetone, tetrahydrofuran, diethyl ether, or ethyl acetate. Quaternary salts can be prepared in generally the same way by reaction of the base with an alkyl sulfate or alkyl halide, for example, methyl sulfate, methyl iodide, ethyl bromide, and propyl iodide.

The inorganic acid salts, especially the hydrochloride salts, are preferred.

The preferred compounds of this invention are those in which

a) $R_1$ is $C_1$-$C_4$ alkoxy, especially methoxy,
b) $R_1$ is hydroxy,
c) $R_2$ is hydrogen,
d) $R_2$ is $C_1$-$C_4$ alkyl, especially methyl,

e) $R_2$ is $C_2$-$C_6$ alkenylmethyl, especially 2-propenyl (allyl),

f) $R_2$ is cyclopropylmethyl,

g) $R_2$ is 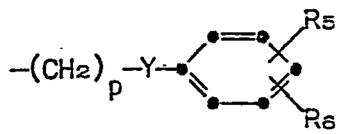 , especially 2-phenylethyl,

h) $R_3$ is hydrogen, and

i) n is 1.

Especially preferred are those compounds wherein $R_1$ is at the 9-position of the molecule.

The bridgehead ring at 4a and 10b has a cis relationship and since both the 4a and 10b carbon atoms are asymmetric, the compounds occur as optical isomer mixtures designated as the cis-dl pair. Formula I is thus intended to comprehend the cis-dl mixture, and the individual enantiomorphs, namely the cis-l and cis-d compounds, since as far as is known, all of the individual isomers and isomer mixtures are useful as psychotropic agents and as analgesic agonists or as analgesic antagonists, albeit large quantitative differences in agonist or antagonistic potency may exist between individual isomers or mixtures. Where $R_3$ is other than hydrogen, an additional chiral center is present; both possible isomers and their mixture are contemplated by this invention.

The novel compounds of this invention are prepared according to scheme I:

Scheme I

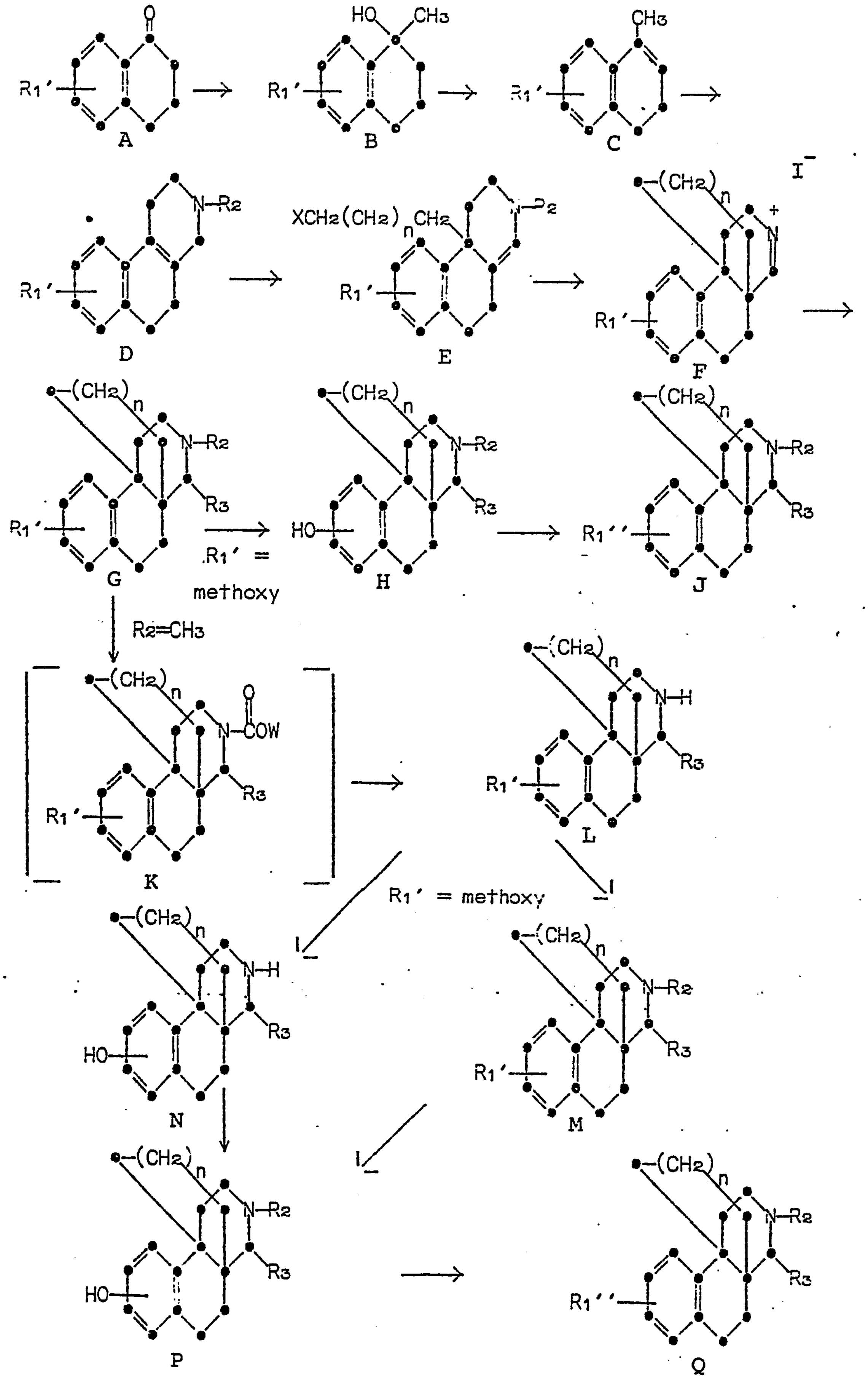

In scheme I, $R_2$, $R_3$ and n are the same as described earlier, $R_1$' is hydrogen or methoxy, $R_1$'' is $C_2$-$C_4$ alkoxy or $C_1$-$C_{12}$ alkanoyloxy, X is chloro, bromo, or iodo, and W is $C_1$-$C_4$ alkyl, benzyl, or phenyl.

A 1-tetralone (A) ($R_1$' is methoxy or hydrogen) is converted to the corresponding $R_1$'-substituted-1-hydroxy-1-methyl-1,2,3,4-tetrahydronaphthalene (B) by treatment with a methyl Grignard reagent in the usual manner. Treatment of the hydroxy intermediate (B) with a dehydrating agent, such as polyphosphoric acid, sulfuric acid, or p-toluenesulfonic acid in a solvent such as benzene, xylene, toluene, or the like with the concurrent removal of water, usually at elevated temperatures, results in the loss of water and the formation of a $R_1$'-substituted-1-methyl-3,4-dihydronaphthalene (C). Aminomethylation of the latter intermediate with two equivalents of formaldehyde, and the appropriate $R_2$-amine gives a $R_1$'-substituted-3-$R_2$-substituted-1,2,3,4,5,6-hexahydrobenz[f]isoquinoline (D) which is the required intermediate for all subsequent transformations.

Treatment of the $R_1$'-substituted-3-$R_2$-substituted-1,2,3,4,5,6-hexahydrobenz[f]isoquinoline (D) with a strong base, such as n-butyl lithium, sodium amide, or methyl lithium, in a compatible solvent, such as ethers, tetrahydrofuran, alkanes, such as hexane, and the like, preferably at low temperatures, for instance 0°C. to -78°C., followed by the addition of the resulting anion to a solution of the desired alkane dihalide $X-CH_2-(CH_2)_n-CH_2-X'$, results in the preparation

of the corresponding $R_1$'-substituted-10b-haloalkyl-substituted-3-$R_2$-substituted-1,2,3,5,6,10b-hexahydrobenz[f]isoquinoline (E). In the formula of the above dihalide, n is 1 or 2 and each of X and X' is independently chloro, bromo, or iodo. Although the reaction will proceed when X and X' are the same, the preferred reactant is one where X and X' are different, especially where one of X and X' is chloro and the other of X and X' is bromo. Thus, the preferred reagents are 1-bromo-3-chloropropane and 1-bromo-4-chlorobutane. When X and X' are different, the resulting compound of structure E will have the halo radical X of lesser atomic weight; eg, if a bromochloroalkane is used, the resulting compound E will be the chloro derivative.

The haloalkyl intermediate E is transformed to the bridged intermediate F by heating in a non-reactive solvent with an alkali metal iodide. This treatment serves to form the propano- or butano-bridged ring and shift the double bond to the iminium species which is complexed with the iodide ion. Suitable non-reactive solvents may include acetonitrile, dioxane, or diglyme. Potassium iodide or sodium iodide may be employed as the alkali metal iodide. The reaction is typically carried out at temperatures ranging from 40°C. to the reflux temperature of the reaction mixture, the latter being preferred to expedite the reaction. Under reflux conditions, the reaction is usually complete within 6 to 48 hours, depending upon the solvent and nature of the alkane dihalide employed.

The iminium iodide intermediate F is transformed into compound G by one of several means, depending upon the nature of the $R_3$ group to be introduced. When the desired product of Formula I is unsubstituted at the 4-position ($R_3$ is hydrogen), intermediate F is reduced by any of a number of methods well known to those skilled in the art. More specifically, the reduction can be accomplished by reaction with hydride reagents such as, for example, sodium borohydride, diborane, lithium aluminum hydride, or sodium bis-(2-methoxyethoxy)aluminum hydride. The hydride reduction reactions are generally carried out in the presence of a suitable solvent, for example, ethers such as diethyl ether, dipropyl ether, tetrahydrofuran, diglyme, or in alcohols such as methanol, ethanol, or isopropanol. Weak acids, such as formic acid or acetic acid, can be added to the reaction mixture if desired, thereby making the reduction more efficient. A preferred reduction method, for example, comprises treating the iminium iodide derivative with sodium borohydride in the presence of a suitable solvent, such as tetrahydrofuran. The amount of reducing agent used can vary over a wide range. Generally, the amount of reducing agent used is in excess of the iminium iodide derivative, normally from about 1 to about 10 molar excess; however, more or less can be used if desired. The reaction is usually carried out at a temperature below about 180°C., although the precise temperature is not critical. Preferably, the reactants are mixed while the temperature is maintained at about 0° to about

20°C. After the reactants have been combined, the temperature can be increased, preferably to a temperature from about 50° to about 150°C. Normally, the reduction is complete within about 1 to about 4 hours. The product can be recovered by making the reaction mixture alkaline, for example by adding an aqueous base such as sodium hydroxide, potassium hydroxide, or ammonium hydroxide, and extracting the aqueous alkaline reaction mixture with a water immiscible organic solvent, such as, diethyl ether, ethyl acetate, or dichloromethane.

The reduction of the iminium iodide compound (F) can alternatively be accomplished by hydrogenation. The hydrogenation is preferably carried out in an unreactive solvent such as, for example, tetrahydrofuran, dioxane, ethanol, methanol, water, or N,N-dimethylformamide, under neutral, acidic, or basic conditions. The particular solvent used is not critical, but preferably the solvent selected is one in which the iminium iodide derivative will be at least partially soluble. The hydrogenation is carried out in the presence of a suitable catalyst such as Raney nickel, platinum oxide, platinum, or palladium on a suitable carrier such as carbon or barium sulfate. The particular reaction conditions are not critical to the process, but generally the hydrogenation is carried out at a temperature of about 25° to about 200°C., with a hydrogen gas pressure of about 20 to about 100 p.s.i.

Alternatively, F may be reacted with a Grignard reagent ($R_3MgX$) or lithium reagent ($R_3Li$)

wherein $R_3$ and X are the same as previously defined, in the usual manner to give the 4-$R_3$-substituted compounds of this invention.

Compounds of structure H are prepared by the cleavage of the methoxy group (G, $R_1$' is methoxy) to the corresponding phenol using known methods such as treatment with hydrobromic acid and acetic acid, or boron tribromide, for example. Such methods of ether cleavage are well known to those in the art; see for example U.S. Patent No. 3,324,139. The phenol compounds are useful as analgesic agents and additionally serve as intermediates to $C_1$-$C_4$ alkoxy and $C_1$-$C_{12}$ acyloxy derivatives (J, $R_1$'' is $C_1$-$C_4$ alkoxy or $C_1$-$C_{12}$ alkanoyloxy). These phenols may be alkylated with $C_1$-$C_4$ alkyl halides in which $C_1$-$C_4$ alkyl and halide are as defined previously, optionally, in the presence of an acid binding agent, such as sodium hydroxide, potassium carbonate, triethylamine, or magnesium hydroxide, to give the appropriate alkoxy substituted-3-$R_2$-substituted product (J, $R_1$'' is $C_1$-$C_4$ alkoxy). Typically, equimolar quantities of the phenol and the alkylating agent are stirred together, preferably in a non-reactive solvent, including amides such as dimethyl formamide or dimethylacetamide, or sulfoxides such as dimethyl sulfoxide, at temperatures from about 0°C. up to the reflux temperature of the solution, preferably at about ambient temperature. The alkanoyl derivatives are similarly prepared by the action of an appropriate $C_1$-$C_{12}$ alkanoyl halide on the phenol in like manner to also provide compounds of structure J ($R_1$'' is $C_1$-$C_{12}$ alkanoyloxy).

Alternatively, the appropriate substituted-1,2,3,4,5,6-hexahydrobenz[f]isoquinolines (G, especially where $R_2$ is methyl) may be dealkylated to the corresponding 3-hydrogen compound (L) by treating the compound with a haloformate to provide a carbamate (K, W is $C_1$-$C_4$ alkyl, benzyl, or phenyl) which gives a secondary amine when treated with a base. This type of reaction is described in more detail by Abdel-Monen and Portoghese, J. Med. Chem., 15, 208 (1972). In particular a haloformate such as, phenyl chloroformate, ethyl bromoformate, or benzyl chloroformate, is reacted with a tertiary amine, for example a 3-methyl isoquinoline derivative in the present case, to afford a carbamate. The reaction is generally carried out in an unreactive solvent such as dichloromethane, chloroform, acetone, or ethyl acetate. The temperature is usually held below about 200° C., and the reaction is substantially complete within about 1 to 5 hours. The product carbamate (K) can be isolated by simply evaporating the reaction solvent, and further purification of the product is generally not needed. The carbamate (K) is converted to the 3-hydrogen compound by the action of a suitable base such as aqueous sodium hydroxide or aqueous potassium carbonate. A suitable organic solvent is normally added to the reaction mixture in order to dissolve the carbamate. Suitable solvents may include alcohols such as ethanol or methanol, or ethers such as dioxane or tetrahydrofuran. The hydrolysis reaction is generally complete within about 12 to 36 hours when carried out at a temperature

of about 50° to about 150°C. The product may be isolated by extracting the aqueous reaction mixture with a suitable solvent such as diethyl ether or methylene chloride. The unsubstituted compound (L) can be further purified by methods such as chromatography, crystallization, distillation, or the like.

These 3-hydrogen compounds (L) are useful analgesic agents and also may be transformed into the corresponding methoxy $R_1$'-substituted-3-$R_2$-substituted-1,2,3,4,5,6-hexahydrobenz[f]isoquinolines (M) in the usual manner, or the methoxy functionality may be cleaved to the phenol (N) using hydrobromic acid/acetic acid. The phenol (N) may then be alkylated at the 3-position with a $R_2$-halide as before to yield (P) and subsequently alkylated at the phenol position with a $R_1$''-halide to give (Q) if desired.

The alkylation of a phenol derivative ($R_1$ is hydroxy) and most of the 3-hydrogen derivatives ($R_2$ is hydrogen) may be performed in the usual manner with the appropriate alkyl halide reagents in the presence of an acid binding agent. The reactants are generally mixed in about equimolar quantities, preferably in an organic solvent. Typical solvents commonly used include amides such as dimethylformamide or dimethylacetamide, or sulfoxides such as dimethyl sulfoxide. Generally, a base is added to the reaction mixture to act as an acid binding agent. Typical bases include sodium bicarbonate, potassium carbonate, magnesium hydroxide, or triethylamine. The alkylation is generally complete after about 1 to about 16 hours when carried out at a temperature of about 20° to 120°C.

The products are isolated by general methods and further purified if desired. If $R_1$ is hydroxy and $R_2$ is hydrogen, the 3-position will be selectively alkylated with one equivalent of halide, although alkylation at the phenol position will occur if more than one equivalent of halide is employed.

The introduction of certain $R_2$ substitutents may additionally be accomplished by treating the 3-hydrogen derivative with an appropriate acyl halide, followed by reduction of the resulting carbamate. This process may be used when there is to be a methylene group adjacent to the nitrogen atom. For instance, ($C_3$-$C_8$ cycloalkyl)methyl compounds may be formed by using a $C_3$-$C_8$ cycloalkyl carboxylic acid halide, followed by reduction. The 2-phenylethyl functionality may be introduced by using a phenylacetyl halide, followed by reduction. Typically, the carboxylic acid chloride is used, usually in the presence of an acid scavenger, such as triethylamine, or sodium bicarbonate, in a compatible solvent, such as benzene, dimethylformamide, or dioxane. The carbamate intermediate may be isolated and reduced to the methylene compound with, for instance, lithium aluminum hydride in tetrahydrofuran. In a similar manner, the $R_2$ substituents in which Y is $-\overset{O}{\overset{\|}{C}}-$ may be transformed into the corresponding alcohol and methylene compounds by partial or total reduction of the ketone.

In Scheme I, 1-tetralone and all four methoxy tetralones (A) are commercially available and thus give

the corresponding hydrogen and methoxy compounds of this invention (G). This provides, through cleavage of the methoxy compounds to the corresponding phenol and subsequent alkylation or acylation, an efficient synthesis to $R_1$''-substituted compounds of this invention. As will be obvious to those skilled in the art, other alkoxy tetralones may be used as starting materials in this sequence to give the corresponding compounds of this invention directly. The hydroxy or acyloxy congeners of (A) cannot be utilized as starting materials because side reactions will occur.

Typical hexahydrobenz[f]isoquinolines provided by this invention include the following:

1,2,3,4,5,6-hexahydro-3-cyclopropylmethyl-8-ethoxy-4a,10b-propanobenz[f]isoquinoline,

1,2,3,4,5,6-hexahydro-3-benzyl-4a,10b-propanobenz[f]isoquinolin-9-ol,

1,2,3,4,5,6-hexahydro-4a,10b-propanobenz[f]-isoquinoline,

1,2,3,4,5,6-hexahydro-9-methoxy-4a,10b-propanobenz[f]isoquinoline,

1,2,3,4,5,6-hexahydro-3,4-diethyl-7-methoxy-4a,10b-butanobenz[f]isoquinoline,

1,2,3,4,5,6-hexahydro-3-methyl-8-acetoxy-4a,10b-butanobenz[f]isoquinoline,

1,2,3,4,5,6-hexahydro-4-methyl-10-n-propoxy-4a,10b-propanobenz[f]isoquinoline,

1,2,3,4,5,6-hexahydro-4a,10b-butanobenz[f]-isoquinoline,

1,2,3,4,5,6-hexahydro-3-(2-phenylethyl)-4-octyl-8-acetoxy-4a,10b-butanobenz[f]isoquinoline,

1,2,3,4,5,6-hexahydro-3-t-butyl-4a,10b-butanobenz[f]isoquinolin-7-ol,

1,2,3,4,5,6-hexahydro-3-cyclopentylmethyl-4-ethyl-4a,10b-propanobenz[f]isoquinoline,

1,2,3,4,5,6-hexahydro-3-octyl-9-methoxy-4a,10b-butanobenz[f]isoquinoline,

1,2,3,4,5,6-hexahydro-7-ethoxy-4a,10b-butanobenz[f]isoquinoline,

1,2,3,4,5,6-hexahydro-3-cyclohexylmethyl-4a,10b-butanobenz[f]isoquinolin-8-ol,

1,2,3,4,5,6-hexahydro-4a,10b-propanobenz-[f]isoquinolin-9-ol,

1,2,3,4,5,6-hexahydro-3-isopropyl-4-hexyl-4a,10b-butanobenz[f]isoquinoline,

1,2,3,4,5,6-hexahydro-3-cycloheptylmethyl-4-butyl-4a,10b-propanobenz[f]isoquinoline,

1,2,3,4,5,6-hexahydro-3-methyl-10-ethoxy-4a,10b-propanobenz[f]isoquinoline,

1,2,3,4,5,6-hexahydro-8-methoxy-4a,10b-propanobenz[f]isoquinoline,

1,2,3,4,5,6-hexahydro-3-(4-methylcyclohexylmethyl)-4a,10b-butanobenz[f]isoquinolin-10-ol,

1,2,3,4,5,6-hexahydro-4-propyl-4a,10b-propanobenz[f]isoquinoline,

1,2,3,4,5,6-hexahydro-4a,10b-butanobenz-[f]isoquinolin-9-ol,

1,2,3,4,5,6-hexahydro-3-(3-butenyl)-4a,10b-butanobenz[f]isoquinoline,

1,2,3,4,5,6-hexahydro-3-methyl-4a,10b-propanobenz[f]isoquinolin-9-ol,

1,2,3,4,5,6-hexahydro-4-methyl-10-isopropoxy-4a,10b-propanobenz[f]isoquinoline,

1,2,3,4,5,6-hexahydro-3-cyclobutylmethyl-4a,10b-propanobenz[f]isoquinolin-10-ol,

1,2,3,4,5,6-hexahydro-3-benzyl-4-octyl-10-formoyl-4a,10b-butanobenz[f]isoquinoline,

1,2,3,4,5,6-hexahydro-3-hexyl-7-(4-butyloctanoyloxy)-4a,10b-propanobenz[f]isoquinoline,

1,2,3,4,5,6-hexahydro-3-methyl-4a,10b-butanobenz[f]isoquinolin-9-ol,

1,2,3,4,5,6-hexahydro-10-methoxy-4a,10b-butanobenz[f]isoquinoline,

1,2,3,4,5,6-hexahydro-3-ethyl-4-heptyl-4a,10b-propanobenz[f]isoquinolin-7-ol,

1,2,3,4,5,6-hexahydro-7-methoxy-4a,10b-propanobenz[f]isoquinoline,

1,2,3,4,5,6-hexahydro-3-propyl-4a,10b-propanobenz[f]isoquinoline,

1,2,3,4,5,6-hexahydro-3,4-dimethyl-7-octanoyloxy-4a,10b-butanobenz[f]isoquinoline,

1,2,3,4,5,6-hexahydro-3-(4-ethylhexyl)-7-butanoyloxy-4a,10b-butanobenz[f]isoquinoline,

1,2,3,4,5,6-hexahydro-3-[2-(3,4-dimethoxyphenoxy)ethyl]-4-methyl-4a,10b-propanobenz[f]isoquinoline,

1,2,3,4,5,6-hexahydro-3-(5-methyl-2-furylmethyl)-7-formoyl-4a,10b-butanobenz[f]isoquinoline,

1,2,3,4,5,6-hexahydro-4-propyl-9-propoxy-4a,10b-propanobenz[f]isoquinoline,

1,2,3,4,5,6-hexahydro-3-methyl-10-lauroyloxy-4a,10b-butanobenz[f]isoquinoline,

1,2,3,4,5,6-hexahydro-3-[2-(3-methyl-2-furylmercapto)ethyl]-4-propyl-4a,10b-butanobenz[f]-isoquinolin-9-ol,

1,2,3,4,5,6-hexahydro-3-cycloheptylmethyl-4-methyl-9-methoxy-4a,10b-butanobenz[f]isoquinoline,

1,2,3,4,5,6-hexahydro-3-(3,4-dichlorobenzyl)-8-propionyloxy-4a,10b-propanobenz[f]isoquinoline,

1,2,3,4,5,6-hexahydro-3-hexyl-4a,10b-propanobenz[f]isoquinolin-7-ol,

1,2,3,4,5,6-hexahydro-3-[2-(4-chloroanilino)-ethyl]-9-acetoxy-4a,10b-butanobenz[f]isoquinoline,

1,2,3,4,5,6-hexahydro-3-[(4-methyl-2-thienyl)-methyl]-4-methyl-4a,10b-propanobenz[f]isoquinolin-8-ol,

1,2,3,4,5,6-hexahydro-3-benzyl-7-butoxy-4a,10b-propanobenz[f]isoquinoline,

1,2,3,4,5,6-hexahydro-3-[3-(4-hydroxyphenyl)-propyl]-4-ethyl-4a,10b-butanobenz[f]isoquinoline,

1,2,3,4,5,6-hexahydro-3-[3-(2-furyl)propyl]-4a,10b-butanobenz[f]isoquinolin-8-ol,

1,2,3,4,5,6-hexahydro-4-heptyl-10-butanoyl-oxy-4a,10b-butanobenz[f]isoquinolin-10-ol,

1,2,3,4,5,6-hexahydro-3-(3-methylbenzyl)-8-decanoyloxy-4a,10b-propanobenz[f]isoquinoline,

1,2,3,4,5,6-hexahydro-3-(3-aminobenzyl)-4-(4-ethylhexyl)-9-acetoxy-4a,10b-propanobenz[f]isoquinoline,

1,2,3,4,5,6-hexahydro-3-[2-(2-thienyl)ethyl]-4a,10b-butanobenz[f]isoquinolin-9-ol,

1,2,3,4,5,6-hexahydro-7-hexanoyloxy-4a,10b-propanobenz[f]isoquinoline,

1,2,3,4,5,6-hexahydro-3-(2-methoxy-4-methylmercaptobenzyl)-4a,10b-butanobenz[f]isoquinolin-7-ol,

1,2,3,4,5,6-hexahydro-3-(3-phenylpropyl)-4-methyl-4a,10b-propanobenz[f]isoquinoline,

1,2,3,4,5,6-hexahydro-3-[3-(4-trifluoromethylphenyl)propyl]-10-methoxy-4a,10b-butanobenz-[f]isoquinoline,

1,2,3,4,5,6-hexahydro-3-(4-fluorobenzyl)-4-isopropyl-7-acetoxy-4a,10b-butanobenz[f]isoquinoline,

1,2,3,4,5,6-hexahydro-3-allyl-7-pentanoyloxy-4a,10b-propanobenz[f]isoquinoline, and

1,2,3,4,5,6-hexahydro-3-[2-(3,4-dimethylphenyl)ethyl]-4a,10b-propanobenz[f]isoquinolin-8-ol.

The compounds of this invention are valuable psychotropic and analgesic agents for treating animals suffering from pain. The analgesic potencies of the compounds of Formula I are comparable to that of meperidine as demonstrated, for instance, in the standard mouse writhing assay. The compounds are not "morphine-like" when tested in mice, and have demonstrated narcotic antagonist-like properties.

The analgesic activity of a number of compounds provided by this invention has been determined in the standard rat-tail jerk assay and the mouse writhing assay.

Mouse Writhing Inhibition Assay

Writhing, characterized by contraction of the abdominal musculature, extension of the hindlegs, and

rotation of the trunk, was induced in Cox standard strain albino male mice. The mice, weighing 18-24 grams, were fasted overnight and given the test compound by gavage in an acacia suspension (5%) sixty minutes before writhing was induced by the intraperitoneal administration of acetic acid (0.60 percent). Each treatment group consisted of 5 mice. The total number of writhes for the treatment group was determined during a 10-minute observation starting 5 minutes after acetic acid administration. Control groups had a total of 40-60 writhes per observation period.

Table I shows typical test results obtained with the claimed compounds. The results in the mouse writhing assay are presented as the effective dose in mg./kg. of the tested compound required to inhibit induced writhing in the test animals by fifty percent ($ED_{50}$). Results are presented in Column II for both subcutaneous (s.c.) and oral (p.o.) administration of the test compound as compared to the reference compound meperidine.

Table I

| Compound of Example No. | Column II Mouse Writhing $ED_{50}$ (mg./kg.) s.c. | p.o. |
|---|---|---|
| 1 | 17 | 23 |
| 2 | 2.4 | 17 |
| 3 | 56 | 66 |
| 4 | 52 | 78 |
| 6 | 60 | >80 |
| 7 | >80 | >80 |
| 8 | 23 | 68 |
| meperidine | 2.8 | 21 |

A further aspect of this invention provides for pharmaceutical formulations containing the compounds having the above formula. Such formulations are useful in the treatment of pain in warm-blooded animals. The formulations contemplated comprise an analgesically-effective dose of a compound of the invention in combination with any of a number of pharmaceutical diluents, excipients and carriers. Typical diluents commonly used in such formulations may include lactose, sucrose, starch, micro-crystalline cellulose, calcium sulfate, or sodium benzoate. Typical formulations will contain from about 1 to about 30 percent by weight of active ingredient. The formulations can be compressed into tablets or encapsulated in gelatin capsules for convenient oral administration. Alternatively, the formulations can be dissolved in sterile water or saline and placed in a suitable vial for convenient intravenous or intramuscular administration.

The compounds of the invention have demonstrated valuable analgesic activity, and therefore are useful in the treatment of pain. The invention thus further provides a method for imparting analgesia in an animal which comprises administering an analgesically effective dose of a compound of the invention. Typical doses commonly administered will range from about 0.5 to about 150 mg. per kg. of animal body weight. A preferred dose will be from about 1.0 to about 50 mg./kg. The method of treatment can be accomplished by administering an analgesic compound of the invention via the oral or parenteral routes. Preferred routes of administration include the oral and intramuscular routes. Subcutaneous administration of the suitably formulated active compounds can also be utilized when desired. As an illustration of the contemplated method of treatment, a formulation comprising about 50 mg. of 1,2,3,4,5,6-hexahydro-9-methoxy-4a,10b-propanobenz[f]isoquinoline hydrochloride dissolved in about 1 ml. of saline is administered from 1 to 4 times daily to a subject suffering from pain and in need of analgesic treatment.

To more fully illustrate the various aspects of this invention, the following non-limiting examples are provided.

Preparation 1

1-hydroxy-1-methyl-7-methoxy-1,2,3,4-tetrahydronaphthalene

A solution of 208.8 g. (1.2 moles) of 7-methoxy-1-tetralone in 1800 ml. of anhydrous ether was added dropwise to a solution of 178.8 g. (1.5 moles) of methyl magnesium bromide in 500 ml. of anhydrous ether. After all of the solution was added, the reaction was heated to reflux for 30 minutes, and then quenched with 250 ml. of a saturated ammonium chloride solution. The resulting layers were separated and the organic layer was washed with water, then with saturated sodium chloride solution, and dried over potassium carbonate. The solution was evaporated to dryness, yielding 198.0 g. of a tan solid. Recrystallization from hexane yielded 189.4 g. of the title product as a tan solid, m.p. about 75-77°C.

Analysis: $C_{12}H_{16}O_2$;

Calc: C, 74.97; H, 8.39;
Found: C, 74.68; H, 8.12.

Preparation 2

1-methyl-7-methoxy-3,4-dihydronaphthalene

A solution of 367.2 g. (1.9 moles) of 1-hydroxy-1-methyl-7-methoxy-1,2,3,4-tetrahydronaphthalene and 4.0 g. of p-toluenesulfonic acid in 4 liters of toluene was heated to reflux overnight with water being collected by means of a Dean Stark trap. The reaction mixture was then washed several times with

water followed by a saturated sodium chloride solution wash. The organic solution was dried over potassium carbonate and evaporated to dryness yielding 345.8 g. of a brown liquid. Vacuum distillation of the liquid at 84-86°C. and 0.1 mm. of Hg yielded 310.1 g. of the title product as a colorless liquid.

Analysis: $C_{12}H_{14}O$;

Calc: C, 82.72; H, 8.10;

Found: C, 83.02; H, 7.98.

## Preparation 3

1,2,3,4,5,6-hexahydro-3-methyl-9-methoxy-benz[f]isoquinoline

A solution of 141.1 g. (0.81 moles) of 1-methyl-7-methoxy-3,4-dihydronaphthalene and 263.0 g. of a 37% solution of formaldehyde (3.24 moles) in 700 ml. of acetic acid was heated to 65-70°C. for 30 minutes with stirring. While maintaining the temperature below 70°C., 125.8 g. (1.86 moles) of methylamine hydrochloride were added and the mixture was stirred at 70°C. for 3 hours. The solution was cooled to room temperature, diluted with 1 liter of ice-water, and washed with 3 x 500 ml. of ether. The aqueous layer was made basic with about 500 ml. of 50% sodium hydroxide, and was then extracted with ether. The organic layer was separated and dried over potassium carbonate and evaporated to dryness yielding 163.8 g. of a yellow oil. Vacuum distillation of the oil at 0.01 mm. of Hg between 150 and 205°C. gave 96.2 g. of the title product as a yellow oil. The hydrochloride salt was made

using etherial hydrogen chloride. Upon evaporation of the solvent and recrystallization from ether/ethanol, the title product hydrochloride was obtained, m.p. about 229-232°C.

Analysis: $C_{15}H_{19}NO \cdot HCl$;

Calc: C, 67.99; H, 7.59; N, 5.27;

Found: C, 67.99; H, 7.39; N, 5.07.

Example 1

1,2,3,4,5,6-hexahydro-3-methyl-9-methoxy-4a,10b-propanobenz[f]isoquinoline hydrochloride

A 1.6M solution of n-butyllithium in hexane (81.9 ml., 0.131 moles) was slowly added to a solution of 30.0 g. of 1,2,3,4,5,6-hexahydro-3-methyl-9-methoxy-benz[f]isoquinoline in 500 ml. of dry tetrahydrofuran at -10°C. This solution was then added to a solution of 67.8 g. (0.432 moles) of 1-bromo-3-chloropropane in 400 ml. of anhydrous diethyl ether at -60 to -70°C. The reaction mixture was then quenched with approximately 500 ml. of a saturated sodium chloride solution and further diluted with 1 liter of diethyl ether. After allowing to warm to approximately 0°C., the aqueous layer was separated and extracted with 2 liters of diethyl ether. The combined ether layers were washed 3 times each with 500 ml. of cold water. The ether was then extracted 3 times each with 600 ml. of cold 1N hydrochloric acid. The acid layers were combined and washed with ether. The aqueous layer was then made basic with 50% sodium hydroxide and extracted with ether. The ether extract was dried over potassium

carbonate and evaporated to dryness at 5-15°C. The resulting orange foam was dissolved in 2 liters of acetonitrile. Fifty-four grams (0.524 moles) of sodium iodide were added to the acetonitrile solution and the mixture was refluxed for 24 hours. The mixture was filtered and then evaporated to dryness. The residue was dissolved in 2.5 L. of ethanol giving a green colored solution. The reaction mixture turned to yellow-orange when 52.0 g. (1.38 moles) of sodium borohydride were added. The mixture was stirred at room temperature for 1 hour and then diluted with 1 liter of water. The solution was extracted 3 times each with 1 liter of diethyl ether. The combined ether extracts were washed several times with water, dried over potassium carbonate, and evaporated to dryness yielding 22.2 g. of a yellow-orange oil. Vacuum distillation of the oil at 0.4 mm. of Hg and 192-220°C. gave 15.0 g. of the title product (free base) as a yellow oil. Two grams of the title product (free base) were converted to the hydrochloride salt in etherial hydrogen chloride which upon crystallization from 100 ml. of ethyl acetate and 150 ml. of ethanol provided 1.1 grams of the title product (hydrochloride salt) as a white solid, m.p. about 285-286°C.

Analysis: $C_{18}H_{25}NO \cdot HCl$;

Calc: C, 70.22; H, 8.51; N, 4.55;

Found: C, 70.18; H, 8.38; N, 4.31.

Example 2

1,2,3,4,5,6-hexahydro-3-methyl-4a,10b-propanobenz[f]isoquinolin-9-ol maleate

A solution of 4.0 grams (15 mmoles) of 1,2,3,4,5,6-hexahydro-3-methyl-9-methoxy-4a,10b-propanobenz[f]isoquinoline in 40 ml. of 48% hydrobromic acid and 40 ml. of acetic acid was heated to reflux overnight. The reaction mixture was then diluted with 400 ml. of water, adjusted to pH 10.1, and extracted into 700 ml. of butanol/toluene (3:1). The organic extract was dried over potassium carbonate and evaporated to dryness yielding 4.2 grams of a tan solid. The solid was crystallized from 120 ml. of ethyl acetate giving 1.75 grams of the title product (free base) as a tan solid, m.p. about 210-215°C. The maleate salt was formed in ethanol which upon crystallization from ethyl acetate/ethanol yielded 0.55 grams of the title product maleate salt as white needles, m.p. about 93-96°C.

Analysis: $C_{17}H_{23}NO \cdot C_4H_4O_4$
Calc.: C, 67.54; H, 7.29; N, 3.75;
Found: C, 67.30; H, 7.03; N, 3.61.

Example 3

1,2,3,4,5,6-hexahydro-9-methoxy-4a,10b-propanobenz[f]isoquinoline hydrochloride

Five grams (18 mmoles) of 1,2,3,4,5,6-hexahydro-3-methyl-9-methoxy-4a,10b-propanobenz[f]isoquinoline were dissolved in 80 ml. of methylene chloride. A solution of 2.9 grams (18 mmoles) of phenylchloroformate in 20 ml. of methylene chloride was added and the reaction was refluxed for 4 hours. The reaction mixture was further stirred overnight at room temperature after which the reaction mixture was evaporated to dryness. The residue was dissolved in 100 ml. of 5% sodium hydroxide and extracted into diethyl ether. The ether solution was washed with water, 10% hydrochloric acid, and water again. After drying over potassium carbonate, the ether solution was evaporated to dryness yielding 7.2 g. of a yellow oil. The oil was dissolved in 50 ml. of a 50% aqueous potassium hydroxide solution and 200 ml. of 2B ethanol. The solution was refluxed for 60 hours. The ethanol was evaporated off and the remaining solution was made acidic with concentrated hydrochloric acid. The aqueous solution was washed with diethyl ether and then made basic with 50% sodium hydroxide. The basic solution was extracted with diethyl ether. The ether layer was dried over potassium carbonate and evaporated to dryness yielding 3.2 grams of a yellow oil. The hydrochloride salt was formed and crystallized from 50 ml. of ethyl acetate and 15 ml. of ethanol, giving 3.0 g. of the title product (hydrochloride) as white needles, m.p. about 204-206°C.

Analysis: $C_{17}H_{23}NO \cdot HCl$;

Calc.: C, 69.49; H, 8.23; N, 4.77;

Found: C, 69.29; H, 7.98; N, 5.04.

## Example 4

1,2,3,4,5,6-hexahydro-4a,10b-propanobenz[f]-isoquinolin-9-ol

A solution of 3.6 g. (14 mmoles) of 1,2,3,4,5,6-hexahydro-9-methoxy-4a,10b-propanobenz[f]isoquinoline in 50 ml. of 48% hydrobromic acid and 50 ml. of acetic acid was heated to reflux overnight. The mixture was diluted with 500 ml. of water and made basic with 50% sodium hydroxide to pH 9.0. After bringing the pH to 10.2 using 1N sodium hydroxide, the solution was extracted into 800 ml. of butanol/toluene (3:1). The organic layer was dried over potassium carbonate and evaporated to dryness yielding 2.5 grams of a yellow solid. Recrystallization from 50 ml. of ethyl acetate and 150 ml. of ethanol yielded 1.55 grams of the title product as a tan solid, m.p. about 240-242°C.

Analysis: $C_{16}H_{21}NO$;

Calc.: C, 78.97; H, 8.70; N, 5.76;

Found: C, 78.74; H, 8.85; N, 5.59.

## Example 5

1,2,3,4,5,6-hexahydro-3-(2-phenylethyl)-4a,10b-propanobenz[f]isoquinolin-9-ol

Phenylacetyl chloride (4.3 g., 28 mmoles) was added to a solution of 2.7 grams (11 mmoles) of 1,2,3,4,5,6-hexahydro-4a,10b-propanobenz[f]isoquinolin-9-ol and 2.8 grams (28 mmoles) of triethylamine in 45 ml. of dimethylformamide. The reaction mixture was heated at 70°C. for 2 hours, and then poured into water. The solution was extracted with diethyl ether.

The ether layer was dried over potassium carbonate and evaporated to dryness yielding 3.3 grams of a tan foam. The foam was dissolved in 40 ml. of dry tetrahydrofuran and the resulting solution was added dropwise to a suspension of 1.2 grams of lithium aluminum hydride in 80 ml. of dry tetrahydrofuran. The reaction was refluxed for 3.5 hours and then quenched with ethyl acetate. The inorganic salts were precipitated with a saturated ammonium chloride solution and the organic layer was decanted. The salts were washed with tetrahydrofuran and the organic layers were combined and evaporated to dryness yielding 1.7 grams of a white solid. The solid was crystallized from 60 ml. of ethyl acetate and 20 ml. of ethanol giving 0.65 grams of the title product as a white solid, m.p. about 214-215°C.

Analysis: $C_{24}H_{29}NO$;

Calc.: C, 82.95; H, 8.41; N, 4.03;

Found: C, 82.71; H, 8.27; N, 3.83.

## Example 6

1,2,3,4,5,6-hexahydro-3-cyclopropylmethyl-4a,10b-propanobenz[f]isoquinolin-9-ol

Following the procedure of Example 5, 1.8 grams (7 mmoles) of 1,2,3,4,5,6-hexahydro-4a,10b-propanobenz[f]isoquinolin-9-ol, 1.8 grams (18 mmoles) of triethylamine, and 1.5 grams (14 mmoles) of cyclopropylcarboxylic acid chloride were heated in a total of 50 ml. of dimethylformamide at 70°C. for 1 1/2 hours. The reaction was worked up affording 1.7 grams

of a yellow solid which was treated with 2.0 grams of lithium aluminum hydride and 190 ml. of dry tetrahydrofuran. After refluxing for 4 hours the reaction was worked up giving 1.1 grams of a white foam. Crystallization from ethyl acetate yielded 0.82 grams of the title product as a white solid, m.p. 176-177°C.

Analysis: $C_{20}H_{27}NO$;

Calc.: C, 80.76; H, 9.15; N, 4.71;

Found: C, 80.96; H, 9.18; N, 4.45.

Example 7

1,2,3,4,5,6-hexahydro-3-(2-propenyl)-4a,10b-propanobenz[f]isoquinolin-9-ol

To a solution of 2.5 grams (10 mmoles) of 1,2,3,4,5,6-hexahydro-4a,10b-propanobenz[f]isoquinolin-9-ol and 1.4 grams (11 mmoles) of allyl bromide in 40 ml. of dimethylformamide was added 1.8 grams (22 mmoles) of sodium bicarbonate. The reaction was brought to reflux for 1 hour, cooled, and poured into water. The aqueous solution was extracted with diethyl ether. The ether was washed several times with water, dried over potassium carbonate, and evaporated to dryness yielding 2.5 grams of a brown foam. The foam was crystallized from 80 ml. of ethyl acetate giving 0.76 grams of the title product as a tan solid, m.p. 149-150°C.

Analysis: $C_{19}H_{25}NO$;

Calc.: C, 80.52; H, 8.89; N, 4.94;

Found: C, 80.28; H, 8.63; N, 4.73.

Example 8

1,2,3,4,5,6-hexahydro-3-methyl-propanobenz-[f]isoquinoline hydrobromide

Following the procedure of Example 1, 11.1 grams (56 mmoles) of 1,2,3,4,5,6-hexahydro-3-methyl-benz[f]isoquinoline, prepared by the method of Preparation 3, were dissolved in 180 ml. of dry tetrahydrofuran. The solution was cooled to -7°C. and 35 ml. (56 mmoles) of a 1.6M solution of n-butyllithium in hexane were added in a dropwise manner. After stirring for 10 minutes this solution was added to a solution of 29 grams (185 mmoles) of 1-bromo-3-chloropropane in 135 ml. of anhydrous diethyl ether at -60°C. The reaction was quenched with the addition of 240 ml. of a saturated sodium chloride solution and 1 liter of diethyl ether was added. The layers were separated, and the ether layer was washed 3 times with 150 ml. each of 1N hydrochloric acid. The acid layer was washed with ether and then made basic with 50% sodium hydroxide. The aqueous layer was extracted with diethyl ether and the ether layer was dried over potassium carbonate and evaporated to dryness at 5-15°C. The resulting yellow foam was dissolved in 2.4 liters of acetonitrile, 24.5 grams of sodium iodide was added, and the mixture was refluxed for 24 hours. After cooling to room temperature, the mixture was filtered and the filtrate was evaporated to dryness yielding 34.9 grams of a green oil. One liter of ethanol was added followed by the addition of 20.6 grams of sodium borohydride. After stirring for 1 hour at room temperature, 500 ml. of water was added and the

solution was extracted with 2 liters of diethyl ether. The ether was washed several times with water, dried over potassium carbonate, and evaporated to dryness yielding 8.5 grams of a yellow brown oil. Vacuum distillation of the oil at 0.1 mm. of Hg and 123-130°C. yielded 5.6 grams of a yellow oil which was determined to be the free base of the title product. A portion of this material was converted to the hydrobromide salt which upon crystallization from ethyl acetate/ethanol, yielded the title product as a white solid, m.p. 258-259°C.

Analysis: $C_{17}H_{23}N \cdot HBr$;

Calc.: C, 63.36; H, 7.51; N, 4.35;

Found: C, 63.13; H, 7.75; N, 3.98.

## Example 9

1,2,3,4,5,6-hexahydro-3-(cyclopropylmethyl)-4a,10b-propanobenz[f]isoquinoline hydrochloride

A. Preparation of 1,2,3,4,5,6-hexahydro-4a,10b-propanobenz[f]isoquinoline

Following the procedure of Example 3, 21.5 g. (89 mmoles) of 1,2,3,4,5,6-hexahydro-3-methyl-4a,10b-propanobenz[f]isoquinoline were reacted with 16.61 g. (106 mmoles) of phenyl chloroformate in 350 ml. of methylene chloride. Hydrolysis was accomplished using 190 ml. of 50% aqueous potassium hydroxide solution and 727 ml. of 2B ethanol. Vacuum distillation of the resulting oil at 105-110°C. and 0.02 mm. of Hg gave 12.26 g. of 1,2,3,4,5,6-hexahydro-4a,10b-propanobenz[f]isoquinoline.

B. Preparation of 1,2,3,4,5,6-hexahydro-3-(cyclopropylmethyl)-4a,10b-propanobenz[f]isoquinoline hydrochloride

Following the procedure of Example 6, 2.27 g. (10 mmoles) of 1,2,3,4,5,6-hexahydro-4a,10b-propanobenz[f]isoquinoline, 2.93 g. of triethylamine, and 2.85 g. of cyclopropyl carboxylic acid chloride were reacted in 42 ml. of dimethylformamide followed by reduction with 1.6 g. of lithium aluminum hydride in 75 ml. of dry tetrahydrofuran. After conversion to the hydrochloride salt and crystallization from hexane/isopropanol, 0.87 g. of the title product were obtained, m.p. about 211-213°C.

Analysis: $C_{20}H_{27}N\cdot HCl$;
Calc.: C, 75.56; H, 8.88; N, 4.41;
Found: C, 75.51; H, 8.90; N, 4.32.

## Example 10

1,2,3,4,5,6-hexahydro-3-(cyclopropylmethyl)-9-methoxy-4a,10b-propanobenz[f]isoquinoline hydrochloride

Following the procedure of Example 6, 2.5 g. (10 mmoles) of 1,2,3,4,5,6-hexahydro-9-methoxy-4a,10b-propanobenz[f]isoquinoline, 2.93 g. of triethylamine, and 2.85 g. of cyclopropyl carboxylic acid chloride were reacted in 42 ml. of dimethylformamide, followed by reduction with 1.6 g. of lithium aluminum hydride in 75 ml. of tetrahydrofuran, conversion to the hydrochloride salt, and crystallization from hexane/isopropanol, to give 1.57 g. of the title product, m.p. about 258.5-260°C.

Analysis: $C_{21}H_{29}NO\cdot HCl$;
Calc.: C, 72.49; H, 8.69; N, 4.03;
Found: C, 72.26; H, 8.55; N, 3.77.

Example 11

1,2,3,4,5,6-hexahydro-3-(2-propenyl)-4a,10b-propanobenz[f]isoquinoline hydrochloride

Following the procedure of Example 7, 2.27 g. (10 mmoles) of 1,2,3,4,5,6-hexahydro-4a,10b-propanobenz-[f]isoquinoline were reacted with 1.24 g. of allyl bromide and 1.20 g. of sodium bicarbonate in 35 ml. of dimethylformamide. Conversion to the hydrochloride salt and crystallization from hexane/isopropanol gave 0.75 g. of the title product, m.p. >225°C. (decomposition).

Analysis: $C_{19}H_{25}N \cdot HCl$;
Calc.: C, 75.10; H, 8.62; N, 4.61;
Found: C, 74.91; H, 8.81; N, 4.59.

Example 12

1,2,3,4,5,6-hexahydro-3-ethyl-4a,10b-propanobenz[f]isoquinolin-9-ol

Following the procedure of Example 7, 0.5 g. of 1,2,3,4,5,6-hexahydro-4a,10b-propanobenz[f]isoquinolin-9-ol, 0.31 g. of ethyl iodide, and 0.26 g. of sodium bicarbonate were reacted in 10 ml. of dimethylformamide. Crystallization from ethyl acetate afforded 320 mg. of the title product, m.p. about 181-183°C.

Analysis: $C_{18}H_{25}NO$;
Calc.: C, 79.66; H, 9.29; N, 5.16;
Found: C, 79.73; H, 9.49; N, 5.05.

## CLAIMS

1. A compound of Formula (I),

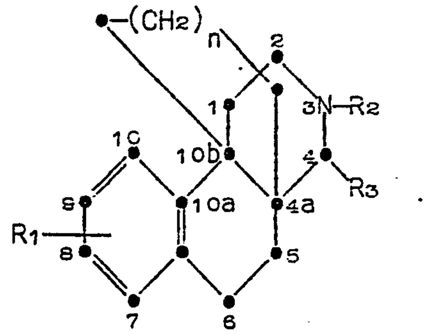

I

or a pharmaceutically-acceptable salt thereof, in which

$R_1$ is hydrogen, hydroxy, $C_1$-$C_4$ alkoxy, or $C_1$-$C_{12}$ alkanoyloxy;

$R_2$ is hydrogen, $C_1$-$C_8$ alkyl, $C_2$-$C_6$ alkenylmethyl, ($C_3$-$C_8$ cycloalkyl)methyl,

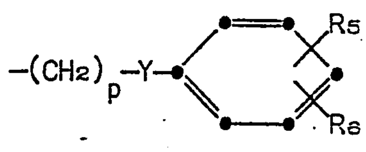

, or

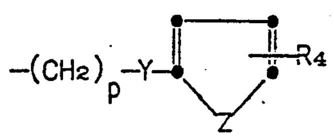

,

in which p is 0, 1, or 2;

Y is $-\overset{O}{\overset{\|}{C}}-$, $-\overset{OH}{\overset{|}{CH}}-$, $-CH_2-$, -O-, -S-, or -NH-; providing that when Y is -O-, -S-, or -NH-, p may only be 2, and providing that when Y is $-\overset{OH}{\overset{|}{CH}}-$, p may not be 0;

Z is O or S;

$R_5$ is $C_1$-$C_4$ alkylthio, nitro, amino, trifluoromethyl, hydroxy, hydrogen, $C_1$-$C_4$ alkyl, $C_1$-$C_4$ alkoxy, or halo;
$R_6$ is hydrogen, $C_1$-$C_4$ alkyl, $C_1$-$C_4$ alkoxy, or halo;
$R_4$ is hydrogen or methyl;
$R_3$ is $C_1$-$C_8$ alkyl or hydrogen; and
n is 1 or 2.

2. A compound as claimed in claim 1 in which $R_1$ is hydroxy or $C_1$-$C_4$ alkoxy, $R_2$ is hydrogen, $C_1$-$C_4$ alkyl, $C_2$-$C_6$ alkenylmethyl, ($C_3$-$C_8$ cycloalkyl)methyl or

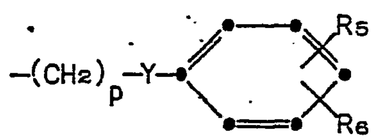

$R_3$ is hydrogen, and n is 1, or a pharmaceutically-acceptable salt thereof.

3. A compound as claimed in claim 1 or 2 in which $R_1$ is methoxy or hydroxy, $R_2$ is methyl, 2-propenyl, cyclopropylmethyl, or 2-phenylethyl, or a pharmaceutically-acceptable salt thereof.

4. 1,2,3,4,5,6-hexahydro-3-methyl-4a,10b-propanobenz[f]isoquinolin-9-ol, or a pharmaceutically-acceptable salt thereof.

5. 1,2,3,4,5,6-hexahydro-3-methyl-9-methoxy-4a,10b-propanobenz[f]isoquinoline, or a pharmaceutically-acceptable salt thereof.

6. 1,2,3,4,5,6-hexahydro-3-(cyclopropylmethyl)-4a,10b-propanobenz[f]isoquin-9-ol, or a pharmaceutically-acceptable salt thereof.

7. A process for preparing a compound of Formula (I), as claimed in any one of claims 1 to 6, or a pharmaceutically-acceptable salt thereof, which comprises:

(a) Reducing a compound of Formula (II),

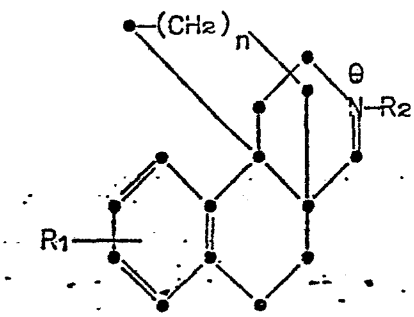

(II)

to form a compound of Formula (I) in which $R_3$ is hydrogen; or,

(b) Reacting a compound of Formula (II) of step (a), in which $R_1$ is hydrogen or $C_1$-$C_4$ alkoxy, with an organometallic reagent to form a compound of Formula (I) in which $R_3$ is $C_1$-$C_8$ alkyl; or,

(c) Hydrolysis or reduction of a compound of Formula (I) in which $R_1$ is $C_1$-$C_4$ alkoxy or $C_1$-$C_{12}$ alkanoyloxy, to form a compound in which $R_1$ is hydroxy; or,

(d) Acylation or alkylation of a compound of Formula (I) in which $R_1$ is hydroxy to form a compound of Formula (I) in which $R_1$ is alkanoyloxy or alkoxy; or,

(e) Treatment of a compound of Formula (I), in which $R_2$ is $-\underset{\overset{\|}{O}}{C}-OW$ where W is $C_1-C_4$ alkyl, benzyl or phenyl, with a base to form a compound of Formula (I) in which $R_2$ is hydrogen; or,

(f) Reacting a compound of Formula (I) in which $R_2$ is hydrogen, with an alkyl halide, or acyl halide followed by reduction, to form a compound of Formula (I) in which $R_2$ is other than hydrogen; and,

(g) if desired, salifying a product of any of reactions (a)-(f).

8. A compound of Formula (I), or a pharmaceutically-acceptable salt thereof, as claimed in any of claims 1 to 6, for use as an analgesic or psychotropic agent in the chemotherapy of warm-blooded animals.

9. A pharmaceutical formulation which comprises as an active ingredient, a compound of Formula (I), or a pharmaceutically-acceptable salt thereof, as claimed in any one of claims 1 to 6, associated with one or more physiologically-acceptable carriers or vehicles therefor.